# EUROPEAN PATENT SPECIFICATION

(11) **EP 1 340 504 B1**
(45) Date of publication and mention of the grant of the patent: **24.05.2006**
(21) Application number: 01978968.4
(22) Date of filing: 31.10.2001
(51) Int. Cl.: A61K 31/7004, A61K 31/7016, A23L 1/30, A23L 2/00, A61P 3/10

(54) **REMEDIES FOR DIABETES**
DIABETESMITTEL
MEDICAMENT CONTRE LE DIABETE

(30) Priority: 15.11.2000 JP 2000347372
(43) Date of publication of application: 03.09.2003
(73) Proprietor: Sanwa Kosan Kabushiki Kaisha, Kashihara-shi, Nara 634-0834 (JP); Fujii, Makoto, Kagoshima-shi, Kagoshima 891-0102 (JP); Hizukuri, Susumu, Kagoshima-shi, Kagoshima 892-0811 (JP)
(72) Inventor: FUJII, Makoto, Kagoshima-shi, Kagoshima 891-0102 (JP); HIZUKURI, Susumu, Kagoshima-shi, Kagoshima 892-0811 (JP); OHSAKI, Shigemitsu, Kashihara-shi, Nara 634-0008 (JP)
(74) Representative: Wakerley, Helen Rachael
(86) International application number: PCT/JP2001/009542
(87) International publication number: WO 2002/040035

(56) References cited:
- EP-A1- 0 560 284
- WO-A1-94/12057
- JP-A- 8 023 973
- US-A- 5 468 734
- NIPPON EIYO SHOKURYO GAKKAISHI vol. 54, no. 3, 2001, pages 155 - 160, XP002906377
- NIPPON EIYO SHOKURYO GAKKAISHI vol. 53, no. 6, 2000, pages 243 - 247, XP002906378
- J. APPL. GLYCOSCI. vol. 46, no. 2, 1999, pages 159 - 165, XP002906379
- KAGAKU TO SEIBUTSU vol. 35, no. 12, 1997, pages 824 - 826, XP002906380

## Description

### Background of the Invention

### 1. Field of the Invention

The present invention relates to remedy for diabetes mellitus in which interaction of L-arabinose with sucrose is utilized, to food or beverage for improvement of diabetes mellitus and also to use of L-arabinose for the treatment of diabetes mellitus.

### 2. Description of the Related Art

Although the great part of saccharides in food is starch, a lot of sucrose is taken as the most preferable sweetener throughout the world. In 1996 for example, 87 g and 185g of sucrose/person/day were consumed in Japan and in the United States, respectively. Ingestion of much sucrose tends to induce hyperglycemia and obesity.

Obesity is a well-known dangerous factor for non-insulin-dependent diabetes mellitus (NIDDM). Many of diabetics are persons suffering from NIDDM. In addition to administration of insulin or administration of an SU agent which accelerates the secretion of insulin, administration of an inhibitor of α-glucosidase, etc. is available for the therapy of NIDDM. The former two agents promote the ingestion of saccharide from blood into cells whereby blood-sugar level is lowered while, in the latter, action of enzyme (glucosidase) which digests and decomposes the saccharide is suppressed and digestion and absorption of the saccharide in small intestine are delayed and/or suppressed to inhibit the ingestion of the saccharide from intestine into blood whereupon a rise in blood-sugar level after meal is suppressed.

On the other hand, L-arabinose has been known to have an action of inhibition of sucrase which is a sucrose-decomposing enzyme in small intestine and it has been recognized that such an action of L-arabinose is utilized to partially inhibit the decomposition of sucrose and to reduce the energy of sucrose. For example, in the Japanese Patent Laid-Open No. Hei-6/65080, there is disclosed that L-arabinose has an action of suppressing the rise of blood-sugar level in mice during 30 to 120 minutes after loading with sucrose. However, that which is disclosed therein is only the above-mentioned action of sucrase-inhibiting of L-arabinose, action of suppression of increase in blood-sugar level after meal and action of suppression of body weight increase caused thereby and, with regard to efficacy of improvement and treatment of diabetes mellitus by lowering the blood-sugar level, it is not made clear.

The present invention has been invented in view of the current state of the prior art as such and its object is to provide a remedy for diabetes mellitus effective to diabetic patient utilizing L-arabinose which resultsin hypoglycemic action,food or beverage for improvement of diabetes mellitus and method for using L-arabinose for the therapy of diabetes mellitus.

In order to achieve such an object, the present inventors have carried out various studies and, as a result, they have unexpectedly found that, although the sole use of L-arabinose shows only a transient suppressive action to hypoglycemia due to its sucrase-inhibiting action, blood-sugar level of mice is drastically lowered and diabetes mellitus is improved and cured when feed in which L-arabinose is mixed in a specific rate with sucrose which is a causing substance for diabetes mellitus is continuously administered to mice KK-A^{y} which is a model for non-insulin-dependent diabetes mellitus whereupon the present invention has been achieved.

### Summary of the Invention

The present invention relates to the use of L-arabinose and sucrose in the manufacture of a medicament or a food/beverage for the treatment of diabetes mellitus.

The remedy for diabetes mellitus is characterized in containing L-arabinose and sucrose (for example, sugar or sugar-containing food or beverage is used as a source for sucrose) as effective ingredients.

The food or beverage for improvement of diabetes mellitus is characterized in containing L-arabinose and sucrose (for example, sugar or sugar-containing foodorbeverage is usedas a source for sucrose) as effective ingredients.

The use of L-arabinose is characterized in that, for the treatment of diabetes mellitus, L-arabinose is taken together with or prior to ingestion of sucrose (for example, sugar or sugar-containing food or beverage is used as a source for sucrose).

### Brief Description of the Drawings

Fig. 1 shows the changes in blood-sugar level (mg/dl) during the raising period of group A and group B for 30 days.
Fig. 2 shows the changes in blood-sugar level (mg/dl) during the raising period of group C and group D for 30 days.

### Detailed Description of the Preferred Embodiments

Remedy for diabetes mellitus and food or beverage for the improvement of diabetes mellitus according to the present invention will be specifically illustrated hereinafter.

With regard to L-arabinose used in the present invention, that which is manufactured by various known methods can be used. For example, there may be used that which is manufactured in high purity and in high efficiency where plant fiber containing L-arabinose as a part of the constituting saccharides is contacted to an acid of low concentration to subject to an acid hydrolysis whereupon L-arabinose is selectively produced as disclosed, for example, in the Japanese Patent Laid-Open No. Hei-11/313700.

With regard to sucrose used in the present invention, there may be used chemically purified sucrose as well as sucrose-containing substance as a source for sucrose such as sugar or sugar-containing food or beverage. There are various sugar products depending upon material, manufacturing method, degree of purification, color, processed form, etc. thereof and any of them may be used in the present invention so far as it contains sucrose.

With regard to the remedy for diabetes mellitus and the food or beverage for improvement of diabetes mellitus according to the present invention, L-arabinose and sucrose maybe ingested separately with time intervals provided that it is possible to result in a state where L-arabinose and sucrose simultaneously are present in intestine. However, since L-arabinose is slowly absorbed from intestinal tract and remains for long time in intestine while sucrose shows quick digestion and absorption, it is effective for the therapy or the improvement of diabetes mellitus that L-arabinose is ingested together with or prior to ingestion of sucrose (sugar or sugar-containing food or beverage). In the remedy for diabetes mellitus or the food or beverage for improvement of diabetes mellitus according to the present invention, it is preferred that L-arabinose is present in a ratio of 0.5~90% by weight or, preferably, 1-50% by weight to sucrose. When the used amount of L-arabinose is less than the above lower limit, it is not preferred because the effect by the joint use of L-arabinose with sucrose is hardly expressed while, when it is more than the above upper limit, it is not preferred in view of economy since an effect due to its amount cannot be expected.

In addition to the use of L-arabinose and sucrose only, the remedy for diabetes mellitus according to the present invention may also be easily manufactured by combining them with known pharmaceutical carriers to give pharmaceutical preparations. Manufacture of such pharmaceutical preparations is usually conducted in such amanner that L-arabinose and sucrose are compounded with pharmaceutically acceptable liquid or solid carrier and, if necessary, solvent, dispersing agent, emulsifier, buffer, stabilizer, filler, binder, disintegrating agent, lubricant, etc. are added thereto to give solid preparations such as tablets, granules, diluted powder, powder and capsules or liquid preparations such as normal liquid, suspension and emulsion. It is also possible to prepare a dried preparation which can be made into liquid by addition of appropriate carrier thereto before use.

It is preferred that the remedy for diabetes mellitus according to the present invention is an oral preparation so as to effectively express the effect by the joint use of L-arabinose and sucrose. With regard to a pharmaceutical carrier for the oral preparation, there may be used, for example, starch, lactose, white sugar, mannitol, carboxymethylcellulose, corn starch and inorganic salt. For the manufacture of the oral preparation, it is also possible to further compound binder, disintegrating agent, surface-active agent, lubricant, fluidity promoter, corrigent, coloring agent, perfume, etc. therewith.

Dose of the remedy for diabetes mellitus according to the present invention may be appropriately determined depending upon dosage form, administering method, object of use and age, body weight and symptom of the patient applied therewith and, although it is not definite, the amount of L-arabinose contained in the preparation is usually from 1 mg to 10 g/kg per day for adults. It goes without saying that the dose varies depending upon various conditions and, in some cases, less dose than above may be sufficient while, in some other cases, more dose than the above range may be necessary. The remedy for diabetes mellitus according to the present invention may be daily ingested by adding to any food or beverage as well as oral administration.

Although there is no particular limitation for the manufacturing method of the food or beverage for improvement of diabetes mellitus according to the present invention, there may be exemplified the manufacture by means of cooking, processing and commonly used manufacturing method for food or beverage provided that the manufactured food or beverage contains L-arabinose and sucrose having an improving action for diabetes mellitus as an effective ingredient. With regard to the food or beverage for improvement of diabetes mellitus according to the present invention, there is no particular limitation for its form so far as sucrose and L-arabinose having an improving action for diabetes mellitus are contained therein, added thereto and/or diluted therein and it covers orally applicable forms such as tablets, granules, capsules, gel and sol.

Although the reason why the joint use of L-arabinose and sucrose in the remedy for diabetes mellitus and the food or beverage for improving diabetes mellitus according to the present invention is effective for treatment and improvement of diabetes mellitus has not been clearly elucidated, it is judged that numbers of specific enterobacteria are increased or activated due to the presence of L-arabinose and sucrose at the same time in intestine and that those bacteria produce a substance which is effective for reducing sugar-blood level by promoting the incorporation of saccharide from blood into cells.

### Examples

Effect of the joint use of L-arabinose and sucrose used in the present invention for the therapy or improvement of diabetes mellitus will be illustrated as hereunder.

### Example 1

Twenty model mice for non-insulin-depending diabetes mellitus showing hyperglycemia (260~270mg/liter) (KK-A^{y}, male, 8 weeks age, Nippon Clair) were preliminarily raised for one week, divided into two groups (group A and group B) and used for the test. Each of the mice was placed in a plastic cage separately and raised by keeping at 23 ± 1°C under a 12-hour bright-and-dark cycle (bright environment from 7 a.m. to 7 p.m. and dark environment from 7 p.m. to 7 a.m. of the next day) where feed and water were freely taken by the mice. Blood-sugar level (concentration of glucose in blood) was measured by a glucose oxidase method after collecting the blood from venous plexus of the eyeground at 9 a.m.

The feed A mentioned in Table 1 (a feed containing α-corn starch, sucrose and cellulose as carbohydrate sources but containing no L-arabinose) was given to the mice of group A while the feed B mentioned in Table 1 (a feed containing α-corn starch, sucrose and cellulose as carbohydrate sources and also containing L-arabinose) was given to the mice of group B and raising was carried out for 30 days.

**Table 1 Composition of the Feed**

| Composition (% by weight) | Feed | | | |
|---|---|---|---|---|
| | A | B | C | D |
| α-Corn starch | 35.85 | 35.85 | 55.85 | 55.85 |
| Sucrose | 20 | 20 | 0 | 0 |
| L-Arabinose ^{*1} | 0 | 2.5 | 0 | 2.5 |
| Cellulose | 5.0 | 2.5 | 5.0 | 2.5 |
| Corn oil | 6 | 6 | 6 | 6 |
| Mixture of minerals ^{*2} | 6 | 6 | 6 | 6 |
| Mixture of vitamins ^{*2} | 2 | 2 | 2 | 2 |
| Choline chloride | 0.15 | 0.15 | 0.15 | 0.15 |
| Casein | 25 | 25 | 25 | 25 |

| | | | | |
|---|---|---|---|---|
| *1: L-Arabinose (purity: 98%) manufactured by Sanwa Dempun Kogyo K. K. | | | | |
| *2: manufactured by Oriental Yeast K. K. | | | | |

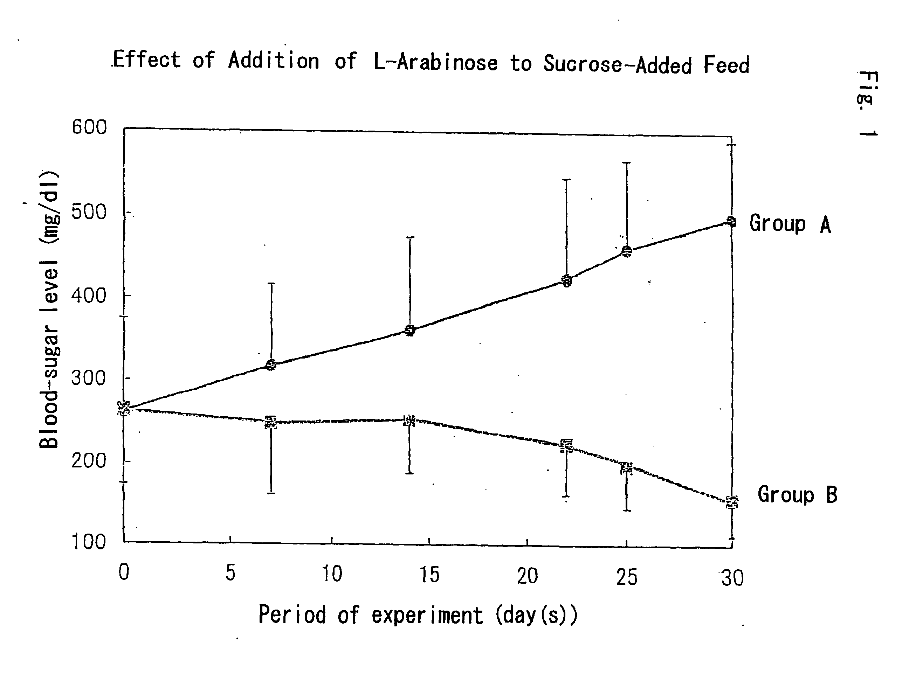

Changes in blood-sugar level (mg/dl) of the groups A and B raised as such for a raising period of 30 days are shown in Fig. 1. As will be apparent from Fig. 1, in the group A where there was given the feed A containing sucrose but containing no L-arabinose, blood-sugar level continued in increasing from the 7th day while, in the group B where there was given the feed B containing sucrose and also containing L-arabinose, lower blood-sugar level than the group A was continuously noted from the 7th day, lower blood-sugar level than that at the start of the experiment was noted after the 21st day and, from the 25th day until 30th day, a continuous decrease in the sugar-blood level was apparently noted whereupon an effect of improving and treating diabetes mellitus was expressed.

Result of Example 1 shows an effect of improving and treating the blood-sugar level is expressed as the joint action by simultaneous administration of sucrose and L-arabinose but, only by way of Example 1, there is still a possibility that addition of L-arabinose only may have the same effect. In order to make the possibility clear, an experiment was carried out in the following Reference Example 1 where no sucrose was added to the feed but α-corn starch was added instead of sucrose.

### Reference Example 1

As same as in Example 1, a feed C mentioned in Table 1 (containing α-corn starch and cellulose as carbohydrate sources but containing neither sucrose nor L-arabinose) was given to the mice of group C while, to the mice of group D, a feed D mentioned in Table 1 (containing α-corn starch and cellulose as carbohydrate sources and also containing L-arabinose but containing no sucrose) was given and raising was carried out for 30 days.

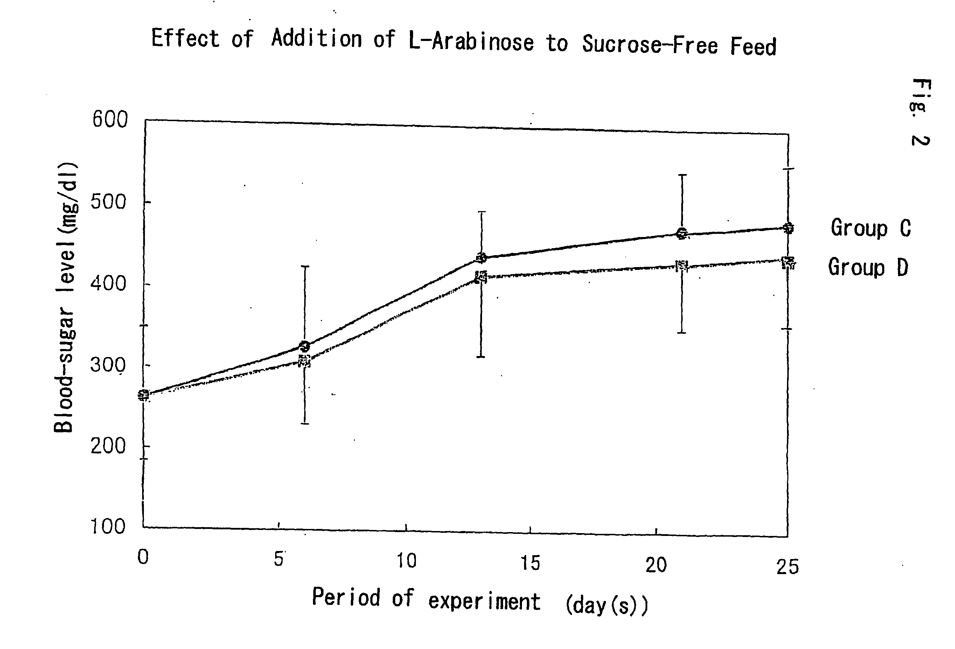

Changes in blood-sugar level (mg/dl) of the groups C and D raised as such for 30 days are shown in Fig. 2. As will be apparent from Fig. 2, mice of both groups C and D continued in increasing the blood-sugar level as same as in the case of the group A of Example 1 during a period of the experiment whereby no improving effect for blood-sugar level was noted. That shows no decrease in blood-sugar level takes place even when only L-arabinose was given without addition of sucrose. Now, results of Example 1 and Reference Example 1 show that high blood-sugar level in model mice for non-insulin-independent diabetes mellitus can be continuously lowered and improved by a simultaneous ingestion of a mixture of sucrose and L-arabinose whereby that is effective for remedy for diabetes mellitus.

Incidentally, it is not always necessary that sucrose and L-arabinose are administered as a mixture thereof but, for example, with regard to sucrose, food or beverage containing sucrose may be ingested separately. In that case, L-arabinose may be ingested either before or after sucrose although the early ingestion is effective.

### Advantage of the Invention

In accordance with the present invention, a continuous decrease in blood-sugar level can be achieved by a joint use of sucrose and L-arabinose and that is very useful as a remedy for diabetes mellitus and as food or beverage for improvement of diabetes mellitus.

## Claims

1. The use of L-arabinose and sucrose in the manufacture of a medicament for the treatment of diabetes mellitus.

2. The use according to claim 1, wherein the medicament is in two parts, the first part being a composition containing L-arabinose and the second part being a composition containing sucrose.

3. The use according to any preceding claim, wherein the medicament is an oral preparation.

4. The use according to any preceding claim, wherein sugar or sugar-containing food or beverage is used as a source for sucrose.

5. The use according to any preceding claim, wherein L-arabinose is present in a ratio of 0.5 to 90% by weight to sucrose.

6. The use according to any preceding claim, wherein L-arabinose is present in a ratio of 1 to 50% by weight to sucrose.

7. The use of L-arabinose and sucrose in the manufacture of a food or beverage for the treatment of diabetes mellitus.

8. The use according to claim 7, wherein L-arabinose is present in a ratio of 0.5 to 90% by weight to sucrose.

9. The use according to claim 7 and 8, wherein L-arabinose is present in a ratio of 1 to 50% by weight to sucrose.

## Patentansprüche

1. Verwendung von L-Arabinose und Saccharose zur Herstellung eines Medikamentes zur Behandlung von Diabetes mellitus.

2. Verwendung nach Anspruch 1, wobei das Medikament aus zwei Teilen besteht, wobei der erste Teil eine Zusammensetzung ist, die L-Arabinose enthält, und der zweite Teil eine Zusammensetzung mit Saccharose ist.

3. Verwendung nach einem der vorherigen Ansprüche, wobei das Medikament ein orales Präparat ist.

4. Verwendung nach einem der vorherigen Ansprüche, wobei Zucker oder ein zuckerhaltiges Lebensmittel oder Getränk als Saccharosequelle verwendet wird.

5. Verwendung nach einem der vorherigen Ansprüche, wobei L-Arabinose in einem Gewichtsanteil von 0,5 bis 90 % der Saccharose vorliegt.

6. Verwendung nach einem der vorherigen Ansprüche, wobei L-Arabinose in einem Gewichtsanteil von 1 bis 50 % der Saccharose vorliegt.

7. Verwendung von L-Arabinose und Saccharose zur Herstellung eines Lebensmittels oder Getränks zur Behandlung von Diabetes mellitus.

8. Verwendung nach Anspruch 7, wobei L-Arabinose in einem Gewichtsanteil von 0,5 bis 90 % der Saccharose vorliegt.

9. Verwendung nach Anspruch 7 und 8, wobei L-Arabinose in einem Gewichtsanteil von 1 bis 50 % der Saccharose vorliegt.

## Revendications

1. Utilisation de L-arabinose et de saccharose dans la fabrication d'un médicament pour le traitement du diabète sucré.

2. L'utilisation selon la revendication 1, dans laquelle le médicament est en deux parties, la première partie étant une composition contenant du L-arabinose et la deuxième partie étant une composition contenant du saccharose.

3. L'utilisation selon l'une quelconque des revendications précédentes, dans laquelle le médicament est une préparation orale.

4. L'utilisation selon l'une quelconque des revendications précédentes, dans laquelle le sucre ou l'aliment ou boisson contenant le sucre est utilisé comme source de saccharose.

5. L'utilisation selon l'une quelconque des revendications précédentes, dans laquelle le L-arabinose est présent selon un rapport de 0,5 à 90% en poids par rapport au saccharose.

6. L'utilisation selon l'une quelconque des revendications précédentes, dans laquelle le L-arabinose est présent selon un rapport de 1 à 50% en poids par rapport au saccharose.

7. L'utilisation de L-arabinose et de saccharose dans la fabrication d'un aliment ou d'une boisson pour le traitement du diabète sucré.

8. L'utilisation selon la revendication 7, dans laquelle le L-arabinose est présent selon un rapport de 0,5 à 90% par rapport au saccharose.

9. L'utilisation selon la revendication 7 et 8, dans laquelle le L-arabinose est présent selon un rapport de 1 à 50% en poids par rapport au saccharose.
